# EUROPÄISCHE PATENTSCHRIFT

(11) **EP 3 102 561 B1**
(45) Veröffentlichungstag und Bekanntmachung des Hinweises auf die Patenterteilung: **13.12.2017**
(21) Anmeldenummer: 15702205.4
(22) Anmeldetag: 26.01.2015
(51) Int. Cl.: C07C 217/30, C07C 217/32, C08G 59/50, C09D 163/10

(54) **AMIN FÜR EMISSIONSARME EPOXIDHARZ-PRODUKTE**
AMINE FOR LOW EMISSION EPOXY RESIN PRODUCTS
AMINE POUR PRODUITS À RÉSINE ÉPOXY PAUVRES EN ÉMISSIONS

(30) Priorität: 07.02.2014 EP 14154363
(43) Veröffentlichungstag der Anmeldung: 14.12.2016
(73) Patentinhaber: Sika Technology AG, 6340 Baar (CH)
(72) Erfinder: KASEMI, Edis, 8046 Zürich (CH); KRAMER, Andreas, 8006 Zürich (CH); STADELMANN, Ursula, 8046 Zürich (CH); BURCKHARDT, Urs, 8049 Zürich (CH)
(74) Vertreter: Sika Patent Attorneys
(86) Internationale Anmeldenummer: PCT/EP2015/051434
(87) Internationale Veröffentlichungsnummer: WO 2015/117846

(56) Entgegenhaltungen:
- WO-A1-2007/060091
- GB-A- 1 258 454

## Beschreibung

### Technisches Gebiet

Die Erfindung betrifft das Gebiet der Amine, der Härter für Epoxidharze, der Epoxidharz-Produkte, sowie deren Verwendung, insbesondere als Beschichtungen, Beläge und Anstriche.

### Stand der Technik

Für Beschichtungszwecke geeignete Epoxidharz-Produkte sollen eine möglichst niedrige Viskosität aufweisen, damit sie bei Umgebungstemperatur gut verarbeitbar und selbstverlaufend sind. Weiterhin sollen sie möglichst schnell und störungsfrei aushärten, auch bei feucht-kalten Bedingungen, und dabei eine ebenmässige Oberfläche ohne Trübungen, Flecken oder Krater ausbilden. Schliesslich soll eine ausgehärtete Beschichtung eine hohe Härte bei geringer Sprödigkeit besitzen, um mechanischer Beanspruchung möglichst gut zu widerstehen. Für optisch anspruchsvolle Anwendungen, beispielsweise Deckbeläge von Fussböden, soll eine Beschichtung ausserdem einen hohen Glanz und eine möglichst geringe Neigung zum Vergilben unter Lichteinfluss aufweisen.

Aus dem Stand der Technik bekannte Härter für Epoxidharz-Beschichtungen enthalten typischerweise Addukte von Polyaminen mit Diepoxiden, insbesondere mit Bisphenol-Flüssigharzen. Solche Addukte ermöglichen eine rasche Aushärtung, sind aber sehr hochviskos, weshalb in den Härtern üblicherweise Verdünner eingesetzt werden. Die Verdünner verbessern die Verarbeitbarkeit, reduzieren die Sprödigkeit der Beschichtung und verbessern die Oberflächenqualität, indem sie das Auftreten von Blushing-Effekten vermindern. Als "Blushing-Effekte" werden bei der Aushärtung auftretende Oberflächenmängel wie Trübungen, Flecken, Rauheit und Klebrigkeit bezeichnet, welche durch Salzbildung ("Blushing") von Aminen mit Kohlendioxid (CO₂) aus der Luft verursacht werden und besonders bei hoher Luftfeuchtigkeit und tiefen Temperaturen auftreten. Die üblicherweise eingesetzten Verdünner, insbesondere Benzylalkohol sowie Glykole und Alkylphenole, sind gegenüber Epoxidharzen bei Raumtemperatur unreaktiv und werden daher bei der Aushärtung nicht in die Harzmatrix eingebaut. Heutzutage werden aber zunehmend emissionsarme Produkte gewünscht, die nach der Aushärtung einen geringen Gehalt von durch Verdampfungs- oder Diffusionsprozesse freisetzbaren Substanzen aufweisen. Für emissionsarme Epoxidharz-Produkte können nicht einbaubare Verdünner deshalb nur in sehr geringer Menge oder gar nicht verwendet werden.

Eine andere Möglichkeit, die Härterkomponente zu verdünnen, besteht im Einsatz von erhöhten Mengen an kleinen Aminen in der Härterkomponente. Solche Amine, wie beispielsweise Dimethylaminopropylamin oder Diethylentriamin, sind aber geruchsintensiv, stark haut- und augenreizend und sensibilisierend, und sie führen verstärkt zu Blushing-Effekten.

Weiterhin können Amin-Addukte von Monoepoxiden, insbesondere Monoglycidylethern, eingesetzt werden, welche gegenüber Amin-Addukten von Diepoxiden eine deutlich verminderte Viskosität aufweisen und deshalb mit weniger Verdünner auskommen. Ein bekanntes niedrigviskoses Addukt wird aus dem aliphatischen Amin 1,5-Diamino-2-methylpentan (MPMD) und Kresylglycidylether erhalten. Dieses ermöglicht verdünnerarme bis -freie Epoxidharz-Beschichtungen, welche weitgehend ohne Blushing-Effekte aushärten. Allerdings sind die Aushärtungsgeschwindigkeit bzw. der Festigkeitsaufbau, die Endhärte und die Viskosität solcher Beschichtungen noch verbesserungsbedürftig. GB 1 258 454 offenbart die Herstellung von Härtern für Epoxidharze durch die Kondensation von tert-Butylphenylglycidylether mit 2,2,4-(2,4,4)Trimethylhexamethylenediamine.

### Darstellung der Erfindung

Aufgabe der vorliegenden Erfindung ist es daher, ein niedrigviskoses und geruchsarmes Amin zur Verfügung zu stellen, das die Formulierung von emissionsarmen Epoxidharzen ermöglicht, die auch bei verhältnismässig tiefen Temperaturen rasch aushärten und dabei qualitativ hochstehende Filme oder Körper von hoher Oberflächenqualität und Festigkeit bilden und unter Lichteinfluss kaum vergilben.

Überraschenderweise wurde gefunden, dass ein Amin der Formel (I) diese Aufgabe sehr gut löst. Es hat einen tiefen Schmelzpunkt, ist überraschend niedrigviskos und geruchsarm und ist in einem einfachen Verfahren aus kommerziell gut zugänglichen und kostengünstigen Substanzen in hoher Reinheit herstellbar.

Bei der Verwendung als Härter ermöglicht das Amin der Formel (I) Epoxidharz-Produkte mit niedriger Viskosität und somit guter Verarbeitbarkeit, einem schnellen Festigkeitsaufbau und hoher Oberflächenqualität, auch bei tiefen Temperaturen. Sowohl im Normklima als auch unter feucht-kalten Bedingungen, beispielsweise 8 °C und 80% relativer Luftfeuchtigkeit, entstehen bei flächiger Anwendung klare Filme von hohem Glanz, welche unter Lichteinfluss kaum vergilben und frei sind von Flecken, Unebenheiten, Strukturen oder Zeichnungen. Das Amin der Formel (I) weist eine sterisch gehinderte primäre Aminogruppe und eine durch die benachbarte Hydroxylgruppe aktivierte sekundäre Aminogruppe auf und ist mit handelsüblichen Epoxid-Flüssigharzen basierend auf Bisphenol-A und/oder -F hervorragend verträglich. Im Vergleich zur Verwendung von ähnlichen Addukten von beispielsweise DETA, MPMD, TMD, 1,3-BAC oder IPDA sind mit dem Amin der Formel (I) Epoxidharz-Produkte mit einer niedrigeren Viskosität und/oder einer besseren Oberflächenqualität erhältlich. Mit dem Amin der Formel (I) sind Epoxidharz-Zusammensetzungen zugänglich, welche auch ohne Verdünner und ohne kleine, geruchsintensive Amine niedrigviskos sind und auch bei tiefen Temperaturen und früher Belastung mit Spritzwasser zu fehlerfreien qualitativ hochwertigen Filmen von hohem Glanz aushärten, welche unter Lichteinfluss kaum vergilben. Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Amin der Formel (I), 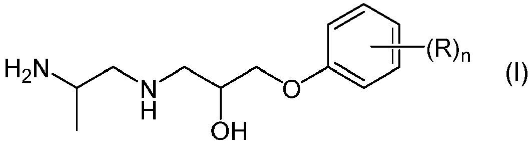 wobei
R für einen Alkyl-, Cycloalkyl-, Aralkyl- oder Alkoxy-Rest mit 1 bis 22 Kohlenstoffatomen, welcher gegebenenfalls ungesättigte Anteile enthält, steht; und
n für 0 oder 1 oder 2 oder 3 steht.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.
Als "primäre Aminogruppe" wird eine NH₂-Gruppe bezeichnet, die an einen organischen Rest gebunden ist und als "sekundäre Aminogruppe" wird eine NH-Gruppe bezeichnet, die an zwei organische Reste, welche auch gemeinsam Teil eines Rings sein können, gebunden ist.
Als "Aminwasserstoff" werden die Wasserstoffatome von primären und sekundären Aminogruppen bezeichnet.
Als "Aminwasserstoff-Equivalentgewicht" wird der Gewichtsanteil eines Härters oder eines Amins pro im Härter oder im Amin vorhandenen Aminwasserstoff bezeichnet.
Als "nicht einbaubarer Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.
Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in DIN EN ISO 3219 beschrieben bestimmt wird.

Die Reste R in der Formel (I) können gleich oder verschieden voneinander sein, falls n für 2 oder 3 steht.
Bevorzugt steht n für 0 oder 1 oder 2, insbesondere für 1. Ein solches Amin der Formel (I) weist eine besonders niedrige Viskosität auf.
Bevorzugt steht R für einen Alkyl- oder Alkoxy-Rest mit 1 bis 18 Kohlenstoffatomen, welcher gegebenenfalls ungesättigte Anteile enthält, besonders bevorzugt für Methyl, tert.Butyl, ungesättigtes Fettalkyl mit 12 bis 18, insbesondere 15, C-Atomen oder Methoxy.
Insbesondere steht R für Methyl, bevorzugt für 2-Methyl.
Am meisten bevorzugt steht n für 1 und R für Methyl, insbesondere 2-Methyl. Diese Amine der Formel (I) sind besonders einfach zugänglich und zeichnen sich durch eine besonders niedrige Viskosität und besonders gute Eigenschaften als Bestandteil von Epoxidharz-Produkten aus, insbesondere eine schnelle Aushärtung und eine hohe Endhärte.
Weiterhin bevorzugt steht n für 1 und R für tert.Butyl, insbesondere 4-tert.Butyl. Diese Amine der Formel (I) ermöglichen ganz besonders schöne Oberflächen. Weiterhin bevorzugt steht n für 1 und R für Fettalkyl mit 15 C-Atomen und ungesättigten Anteilen, insbesondere in 3-Stellung. Diese Amine der Formel (I) sind besonders niedrigviskos und ermöglichen Epoxidharz-Produkte mit eher weichen Eigenschaften.

Das Amin der Formel (I) ist bevorzugt erhältlich aus der Umsetzung von 1,2-Propylendiamin mit einem Arylmonoglycidylether. Es kann auch als Addukt von 1,2-Propylendiamin mit dem Arylmonoglycidylether bezeichnet werden.
Als Arylmonoglycidylether bevorzugt sind die Glycidylether von Phenol, Kresol, Guajacol, 4-Methoxyphenol, tert.Butylphenol oder Cardanol. Als Cardanol wird dabei ein Destillat aus Cashewnussschalen-Öl bezeichnet, welches als Hauptbestandteil 8,11,14-Pentadecatrienylphenol enthält.
Besonders bevorzugte Arylmonoglycidylether sind alle isomeren Kresylglycidylether und beliebige Mischungen davon, insbesondere 2-Kresylglycidylether. Bevorzugt wird ein kommerziell erhältlicher Kresylglycidylether eingesetzt, insbesondere Araldite® DY-K (von Huntsman), Polypox™ R6 (von Dow), Heloxy™ KR (von Momentive) oder Erisys® GE-10 (von CVC Spec. Chem.).

In einem bevorzugten Herstellverfahren wird 1,2-Propylendiamin mit einem Arylglycidylether so umgesetzt, dass pro mol Arylglycidylether mindestens ein mol 1,2-Propylendiamin vorhanden ist.
In einem besonders bevorzugten Herstellverfahren ist pro mol Arylglycidylether mehr als ein mol 1,2-Propylendiamin vorhanden und nicht reagiertes 1,2-Propylendiamin wird nach der Umsetzung abdestilliert, bevorzugt durch Dünnfilm- oder insbesondere Dünnschicht-Destillation.
Bevorzugt ist ein Herstellverfahren mit einem Molverhältnis 1,2-Propylendiamin /Arylglycidylether im Bereich von 1.1 bis 5, besonders bevorzugt 1.5 bis 4, und nachfolgender destillativer Entfernung von unreagiertem 1,2-Propylendiamin.

Ein auf diese Weise hergestelltes Amin der Formel (I) zeichnet sich durch eine besonders hohe Reinheit und eine besonders niedrige Viskosität aus und ermöglicht Epoxidharz-Produkte mit einer besonders guten Verarbeitbarkeit und besonders schönen Oberflächen.
Die Umsetzung wird bevorzugt durch langsames Zudosieren von Arylmonoglycidylether zu vorgelegtem 1,2-Propylendiamin durchgeführt, wobei die Temperatur der Reaktanden bevorzugt im Bereich von 40 bis 120 °C, insbesondere 50 bis 110 °C, gehalten wird.

Das Reaktionsprodukt aus dieser Herstellung kann neben dem Amin der Formel (I) Anteile weiterer Aminaddukte enthalten, insbesondere des Amins der Formel (II) und/oder des Amins der Formel (III). Aufgrund der unterschiedlichen Reaktivität der beiden Aminogruppen von 1,2-Propylendiamin und des bei der Umsetzung bevorzugt eingesetzten Überschusses an 1,2-Propylendiamin gegenüber dem Arylmonoglycidylether wird nach der destillativen Entfernung des überschüssigen 1,2-Propylendiamins jedoch typischerweise sehr reines Amin der Formel (I) erhalten. 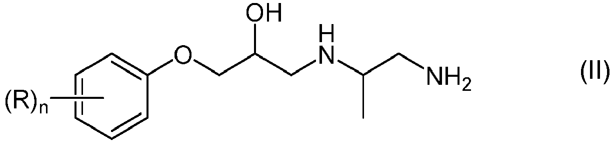 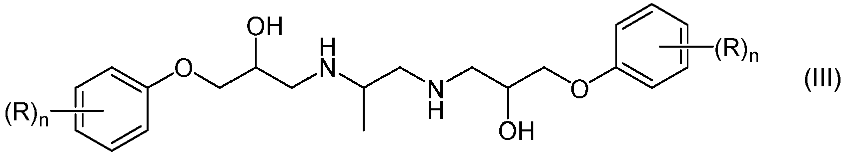 In den Formel (II) und (III) weisen R und n die bereits genannten Bedeutungen auf.

Das Amin der Formel (I) weist sehr vorteilhafte Eigenschaften auf. Es ist niedrigviskos und weist nur einen schwachen Amingeruch auf. Sein Schmelzpunkt ist tief genug, dass es auch bei Wintertemperaturen im flüssigen Zustand gehandhabt und transportiert werden kann, ohne dass die Gefahr des Auskristallisierens besteht.Es verfügt über eine sterisch gehinderte primäre Aminogruppe und eine beta-Hydroxy-substituierte sekundäre Aminogruppe. Dadurch ist es besonders geeignet als Härter für Epoxidharze, wo es niedrigviskose Zusammensetzungen mit sehr schönen Oberflächen ermöglicht, welche auch bei feucht-kalten Bedingungen schnell und ohne Blushing-bedingte Fehler zu hoher Endhärte aushärten.

Ein weiterer Gegenstand der Erfindung ist die Verwendung mindestens eines Amins der Formel (I) in einem Härter für Epoxidharze.
Das Amin der Formel (I) weist dabei den Vorteil auf, dass es nur wenig flüchtig, geruchsarm und niedrigviskos genug ist, um auch ohne den Einsatz von Lösemitteln oder Verdünnern gut verarbeitbare Epoxidharz-Produkte zu ermöglichen. Mit einem Amin der Formel (I) sind niedrigviskose Epoxidharz-Zusammensetzungen mit sehr schönen Oberflächen erhältlich, welche auch bei feucht-kalten Bedingungen schnell und vollständig und ohne Blushing-bedingte Fehler aushärten und unter Lichteinfluss kaum vergilben.

Das Amin der Formel (I) kann allein oder zusammen mit andern Aminen und/ oder Beschleunigern und gegebenenfalls weiteren Substanzen als Härter für Epoxidharze verwendet werden.
Bevorzugt enthält der Härter das Amin der Formel (I) in einer solchen Menge, dass 5 bis 100%, bevorzugt 10 bis 90%, besonders bevorzugt 15 bis 80%, insbesondere 15 bis 70%, der gegenüber Epoxidgruppen reaktiven Aminwasserstoffe im Härter vom Amin der Formel (I) stammen. Ein solcher Härter weist eine gute Balance zwischen niedriger Viskosität und schneller Aushärtung auf und ermöglicht Epoxidharz-Produkte mit schönen Oberflächen und hohen Festigkeiten.

Der Härter enthält neben dem Amin der Formel (I) bevorzugt mindestens ein weiteres Polyamin mit mindestens zwei gegenüber Epoxidgruppen aktiven Aminwasserstoffen.
Dafür geeignet sind insbesondere die folgenden Polyamine:
- aliphatische, cycloaliphatische oder arylaliphatische primäre Diamine, insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis-(4-aminocyclohexyl)methan, Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1 -Amino-3-aminomethyl-3,5,5-trimethylcyclohexan (= Isophorondiamin oder IPDA), 2- und 4-Methyl-1,3-diaminocyclohexan und Mischungen davon, 1,3-Bis(aminomethyl)-cyclohexan (1,3-BAC), 1,4-Bis(aminomethyl)cyclohexan, 2,5(2,6)-Bis(aminomethyl)bicyclo[2.2.1]heptan (NBDA), 3(4),8(9)-Bis(aminomethyl)tricyclo-[5.2.1.0^{2,6}]decan (TCD-Diamin), 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan oder 1,3-Bis(aminomethyl)benzol (MXDA);
- tertiäre Aminogruppen aufweisende Polyamine mit zwei oder drei primären aliphatischen Aminogruppen, insbesondere N,N'-Bis(aminopropyl)-piperazin, N,N-Bis(3-aminopropyl)methylamin, N,N-Bis(3-aminopropyl)ethylamin, N,N-Bis(3-aminopropyl)propylamin, N,N-Bis(3-aminopropyl)cyclohexylamin, N,N-Bis(3-aminopropyl)-2-ethyl-hexylamin, Tris(2-aminoethyl)amin, Tris(2-aminopropyl)amin, Tris(3-aminopropyl)amin, oder die Produkte aus der doppelten Cyanoethylierung und nachfolgender Reduktion von Fettaminen, welche abgeleitet sind von natürlichen Fettsäuren, wie N,N-Bis(3-aminopropyl)dodecylamin oder N,N-Bis(3-aminopropyl)talgalkylamin, erhältlich als Triameen® Y12D oder Triameen® YT (von Akzo Nobel);
- Ethergruppen-haltige aliphatische primäre Polyamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin und höhere Oligomere dieser Diamine, Bis(3-aminopropyl)polytetrahydrofurane und andere Polytetrahydrofurandiamine, cycloaliphatische ethergruppenhaltige Diamine aus der Propoxylierung und nachfolgenden Aminierung von 1,4-Dimethylolcyclohexan, erhältlich insbesondere als Jeffamine® RFD-270 (von Huntsman), oder Polyoxyalkylendi- oder -triamine, welche typischerweise Produkte aus der Aminierung von Polyoxyalkylendi- und -triolen darstellen und beispielsweise erhältlich sind unter dem Namen Jeffamine® (von Huntsman), unter dem Namen Polyetheramine (von BASF) oder unter dem Namen PC Amine® (von Nitroil), insbesondere Jeffamine® D-230, Jeffamine® D-400, Jeffamine® D-2000, Jeffamine® D-4000, Jeffamine® T-403, Jeffamine® T-3000, Jeffamine® T-5000, Jeffamine® EDR-104, Jeffamine® EDR-148 und Jeffamine® EDR-176, sowie entsprechende Amine von BASF oder Nitroil;
- primäre Diamine mit sekundären Aminogruppen, wie insbesondere 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin oder N,N'-Bis(3-amino-1-ethylpropyl)-2-methyl-1,5-pentandiamin;
- Polyamine mit einer primären und mindestens einer sekundären Aminogruppe, wie insbesondere N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, N-(2-Aminoethyl)piperazin, N-Methyl-1,3-propandiamin, N-Butyl-1,3-propandiamin, N-(2-Ethylhexyl)-1,3-propandiamin, N-Cyclohexyl-1,3-propandiamin, 3-Methylamino-1-pentylamin, 3-Ethylamino-1-pentylamin, 3-Cyclohexylamino-1-pentylamin, Fettdiamine wie N-Cocoalkyl-1,3-propandiamin, Produkte aus der Michael-artigen Additionsreaktion von primären aliphatischen Diaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1, weiterhin Produkte aus der partiellen reduktiven Alkylierung von primären Polyaminen mit Aldehyden oder Ketonen, insbesondere N-Monoalkylierungsprodukte der bereits erwähnten Polyamine mit zwei primären Aminogruppen, insbesondere von 1,6-Hexandiamin, MPMD, 1,3-BAC, 1,4-Bis(aminomethyl)-cyclohexan, MXDA, BHMT, DETA, TETA, TEPA, DPTA, N3-Amin oder N4-Amin, wobei als Alkylgruppe Benzyl, Isobutyl, Hexyl und 2-Ethylhexyl bevorzugt sind, sowie weiterhin partiell styrolisierte Polyamine, insbesondere das kommerziell erhältliche Gaskamine® 240 (von Mitsubishi Gas Chemical);
- sekundäre Diamine, wie insbesondere N,N'-Dialkylierungsprodukte der bereits erwähnten Polyamine mit zwei primären Aminogruppen, insbesondere N,N'-Dialkylierungsprodukte von 1,6-Hexandiamin, MPMD, 1,3-BAC, 1,4-Bis-(aminomethyl)cyclohexan, MXDA, BHMT, DETA, TETA, TEPA, DPTA, N3-Amin oder N4-Amin, wobei als Alkylgruppen 2-Phenylethyl, Benzyl, Isobutyl, Hexyl oder 2-Ethylhexyl bevorzugt sind.

Als Polyamin mit mindestens zwei gegenüber Epoxidgruppen aktiven Aminwasserstoffen bevorzugt sind primäre Diamine und/oder Polyamine mit mindestens einer sekundäre Aminogruppe.

Ein Härter mit mindestens einem primären Diamin ermöglicht Epoxidharz-Produkte mit einer besonders niedrigen Viskosität und/oder einem besonders schnellen Festigkeitsaufbau.
In einer bevorzugten Ausführungsform enthält der Härter neben dem Amin der Formel (I) mindestens ein primäres Diamin ausgewählt aus der Gruppe bestehend aus 2,2,4- und 2,4,4-Trimethylhexamethylendiamin (TMD), 1-Amino-3-aminomethyl-3,5,5-trimethylcyclohexan (IPDA), 1,3-Bis(aminomethyl)benzol (MXDA), 1,3-Bis(aminomethyl)cyclohexan (1,3-BAC), 1,4-Bis(aminomethyl)-cyclohexan, Bis(4-aminocyclohexyl)methan, Bis(4-amino-3-methylcyclohexyl)-methan, 2,5(2,6)-Bis(aminomethyl)bicyclo[2.2.1]heptan (NBDA), 3(4),8(9)-Bis-(aminomethyl)tricyclo[5.2.1.0^{2,6}]decan (TCD-Diamin) und Ethergruppen-haltigen Polyaminen mit einem mittleren Molekulargewicht bis zu 500 g/mol.
Diese Polyamine sind kommerziell erhältlich und ermöglichen Härter, welche frei sind von besonders leichtflüchtigen Aminen.

Davon besonders bevorzugt sind die Ethergruppen-haltigen Polyamine, insbesondere Polyoxypropylendiamine wie insbesondere Jeffamine® D-230 (von Huntsman) oder entsprechende Amine von BASF oder Nitroil, sowie cycloaliphatische ethergruppenhaltige Diamine aus der Propoxylierung und nachfolgenden Aminierung von 1,4-Dimethylolcyclohexan, insbesondere Jeffamine® RFD-270 (von Huntsman). Die Ethergruppen-haltigen Polyamine ermöglichen Epoxidharz-Beschichtungen mit besonders niedriger Viskosität, schneller Aushärtung und hoher Schlagzähigkeit.
Davon besonders bevorzugt ist weiterhin IPDA. Dieses cycloaliphatische Amin ermöglicht Epoxidharz-Produkte mit sehr schönen Oberflächen, welche besonders witterungs- und chemikalienbeständig sind.
Davon besonders bevorzugt ist weiterhin 1,3-BAC. Dieses cycloaliphatische Amin ermöglicht Epoxidharz-Produkte mit einem besonders schnellen Festigkeitsaufbau und hoher Witterungs- und Chemikalienbeständigkeit.
Davon besonders bevorzugt ist weiterhin MXDA. Dieses arylaliphatische Amin ermöglicht Epoxidharz-Produkte mit einem besonders schnellen Festigkeitsaufbau und besonders schönen Oberflächen.

Ein Härter, der neben dem Amin der Formel (I) mindestens ein weiteres Polyamin mit mindestens einer sekundären Aminogruppe enthält, ermöglicht Epoxidharz-Produkte mit ganz besonders niedriger Viskosität und besonders hoher Schlagzähigkeit.
Bevorzugt als Polyamin mit mindestens einer sekundären Aminogruppe sind N-monoalkylierte und N,N'-dialkylierte primäre Polyamine und Mischungen davon, wie sie insbesondere durch reduktive Alkylierung von primären Polyaminen mit Aldehyden oder Ketonen und Wasserstoff erhalten werden. Dabei sind Aldehyde gegenüber Ketonen bevorzugt. Bevorzugt sind solche Polyamine, bei welchen 50 bis 100%, bevorzugt 50 bis 80%, insbesondere 50 bis 65%, der ursprünglich primären Aminogruppen als sekundären Aminogruppen vorliegen. Weitere bevorzugte Polyamine mit mindestens einer sekundären Aminogruppe sind Produkte aus der partiellen Styrolisierung von primären Diaminen, insbesondere Gaskamine® 240 (von Mitsubishi Gas Chemical).

In einer bevorzugten Ausführungsform enthält der Härter neben dem Amin der Formel (I) mindestens ein Polyamin mit mindestens einer sekundären Aminogruppe ausgewählt aus der Gruppe bestehend aus N-monoalkyliertem 1,6-Hexandiamin, N,N'-dialkyliertem 1,6-Hexandiamin, N-monoalkyliertem 1,5-Diamino-2-methylpentan, N,N'-dialkyliertem 1,5-Diamino-2-methylpentan, N-monoalkyliertem 1,3-Bis(aminomethyl)cyclohexan, N,N'-dialkyliertem 1,3-Bis-(aminomethyl)cyclohexan, N-monoalkyliertem 1,4-Bis(aminomethyl)cyclohexan, N,N'-dialkyliertem 1,4-Bis(aminomethyl)cyclohexan, N-monoalkyliertem 1,3-Bis(aminomethyl)benzol, N,N'-dialkyliertem 1,3-Bis(aminomethyl)benzol, N-monoalkyliertem BHMT, N,N'-dialkyliertem BHMT, N-monoalkyliertem DETA, N,N'-dialkyliertem DETA, N-monoalkyliertem TETA, N,N'-dialkyliertem TETA, N-monoalkyliertem TEPA, N,N'-dialkyliertem TEPA, N-monoalkyliertem DPTA, N,N'-dialkyliertem DPTA, N-monoalkyliertem N3-Amin, N,N'-dialkyliertem N3-Amin, N-monoalkyliertem N4-Amin und N,N'-dialkyliertem N4-Amin, wobei die Alkylgruppen jeweils ausgewählt sind aus der Gruppe bestehend aus Benzyl, 2-Phenylethyl, Isobutyl, Hexyl und 2-Ethylhexyl. Dabei sind beliebige Kombinationen zwischen den genannten Aminen und den genannten Alkylgruppen möglich.
Diese Polyamine ermöglichen Epoxidharz-Produkte mit besonders niedriger Viskosität, hoher Oberflächenqualität und besonders hoher Schlagzähigkeit. Davon bevorzugt sind mono- und/oder dibenzyliertes 1,3-Bis(aminomethyl)-benzol, mono- und/oder di-2-ethylhexyliertes 1,3-Bis(aminomethyl)benzol und partiell styrolisiertes 1,3-Bis(aminomethyl)benzol, wie insbesondere Gaskamine® 240 (von Mitsubishi Gas Chemical). Diese arylaliphatischen Polyamine sind besonders gut verträglich mit handelsüblichen Epoxidharzen und ermöglichen Epoxidharz-Produkte mit schnellem Festigkeitsaufbau und sehr hoher Oberflächenqualität.

Es kann vorteilhaft sein, wenn im Härter neben dem Amin der Formel (I) sowohl mindestens ein primäres Diamin als auch mindestens ein Polyamin mit mindestens einer sekundären Aminogruppe vorhanden sind. Solche Härter ermöglichen Epoxidharzprodukte mit besonders niedriger Viskosität, schnellem Festigkeitsaufbau, hoher Härte und hoher Schlagzähigkeit. Besonders bevorzugt sind Kombinationen von Ethergruppen-haltigen Polyaminen mit N-monoalkylierten und/oder N,N'-dialkylierten primären Diaminen.

Ein ganz besonders bevorzugter Härter enthält nebem dem Amin der Formel (I) mindestens ein Ethergruppen aufweisendes Diamin und gegebenenfalls ein weiteres primäres Diamin. Bevorzugt als Ethergruppen aufweisendes Diamin sind Jeffamine® D-230 von Huntsman oder ein entsprechendes Amin von BASF oder Nitroil und Jeffamine RFD-270 von Huntsman, insbesondere Jeffamine® D-230 von Huntsman oder ein entsprechendes Amin von BASF oder Nitroil.
Dabei stammen bevorzugt
10 bis 90%, besonders bevorzugt 15 bis 80 %, insbesondere 15 bis 70%, der gegenüber Epoxidgruppen reaktiven Aminwasserstoffe im Härter vom Amin der Formel (I), und
10 bis 60%, bevorzugt 10 bis 50%, insbesondere 20 bis 50%, der gegenüber Epoxidgruppen reaktiven Aminwasserstoffe im Härter vom Ethergruppen aufweisenden Diamin.
Ein solcher Härter weist bevorzugt einen Gehalt an Amin der Formel (I) von mindestens 5 Gewichts-%, bevorzugt mindestens 10 Gewichts-%, insbesondere mindestens 20 Gewichts-%, auf.
Ein solcher Härter weist bevorzugt einen Gehalt an Amin der Formel (I) von weniger als 90 Gewichts-%, bevorzugt weniger als 85 Gewichts-%, insbesondere weniger als 80 Gewichts-%, auf.

Ein weiterer ganz besonders bevorzugter Härter enthält nebem dem Amin der Formel (I) mindestens ein Ethergruppen aufweisendes Diamin und mindestens ein Polyamin mit mindestens einer sekundären Aminogruppe.
Dabei stammen bevorzugt
15 bis 80, insbesondere 15 bis 70%, der gegenüber Epoxidgruppen reaktiven Aminwasserstoffe im Härter vom Amin der Formel (I),
15 bis 60% der gegenüber Epoxidgruppen reaktiven Aminwasserstoffe im Härter vom Ethergruppen aufweisenden Diamin, und
10 bis 35%, bevorzugt 10 bis 20%, der gegenüber Epoxidgruppen reaktiven Aminwasserstoffe im Härter vom Polyamin mit mindestens einer sekundären Aminogruppe.

Der Härter enthält bevorzugt weiterhin mindestens einen Beschleuniger. Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; weiterhin tertiäre Amine wie insbesondere 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiären Amine, quaternäre Ammoniumsalze, wie insbesondere Benzyltrimethylammoniumchlorid, Amidine wie insbesondere 1,8-Diazabicyclo[5.4.0]undec-7-en, Guanidine wie insbesondere 1,1,3,3-Tetramethylguanidin, Phenole, insbesondere Bisphenole, Phenol-Harze, und Mannich-Basen wie insbesondere 2-(Dimethylaminomethyl)phenol, 2,4,6-Tris(dimethylaminomethyl)phenol und Polymere aus Phenol, Formaldehyd und N,N-Dimethyl-1,3-propandiamin, Phosphite wie insbesondere Di- und Triphenylphosphite, sowie Mercaptogruppen aufweisende Verbindungen.
Bevorzugte Beschleuniger sind Säuren, tertiäre Amine oder Mannich-Basen. Besonders bevorzugt sind Salicylsäure und/oder 2,4,6-Tris(dimethylaminomethyl)phenol.

Der Härter enthält bevorzugt keine Amine mit hoher Flüchtigkeit. Bevorzugt ist der Härter frei von 1,2-Propylendiamin oder enthält nur Spuren davon, insbesondere weniger als 0.1 Gewichts-%. Weiterhin bevorzugt ist der Härter frei von Dimethylaminopropylamin und anderen Aminen mit vergleichbar hoher Flüchtigkeit.

In einer bevorzugten Ausführungsform ist der Härter weitgehend frei von Aminen mit einem Molekulargewicht unterhalb von 120 g/mol, insbesondere unterhalb von 150 g/mol, besonders bevorzugt unterhalb von 180 g/mol. Bevorzugt enthält der Härter weniger als 2 Gewichts-%, insbesondere weniger als 1 Gewichts-%, Amine mit einem Molekulargewicht unterhalb von 120 g/mol, insbesondere unterhalb von 150 g/mol, besonders bevorzugt unterhalb von 180 g/mol. Ein solcher Härter ist toxikologisch besonders vorteilhaft und ermöglicht bei flächiger Anwendung besonders schöne Oberflächen.

Der Härter kann weiterhin mindestens einen nicht-einbaubaren Verdünner enthalten. Als nicht-einbaubare Verdünner geeignet sind insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso®-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide. Bevorzugte nicht-einbaubare Verdünner sind Benzylalkohol, Dodecylphenol, tert.Butylphenol, styrolisiertes Phenol, ethoxyliertes Phenol oder phenolgruppenhaltige aromatische Kohlenwasserstoffharze, insbesondere die Novares®-Typen LS 500, LX 200, LA 300 und LA 700 (von Rütgers).

Der Härter enthält bevorzugt keinen oder nur einen geringen Gehalt an nicht einbaubaren Verdünnern, besonders bevorzugt weniger als 25 Gewichts-%, insbesondere weniger als 10 Gewichts-% und am meisten bevorzugt weniger als 5 Gewichts-%. Insbesondere werden dem Härter keine nicht einbaubaren Verdünner zugesetzt.

Der Härter kann weiterhin aromatische Polyamine enthalten, insbesondere mund p-Phenylendiamin, 4,4'-, 2,4' und 2,2'-Diaminodiphenylmethan, 3,3'-Dichloro-4,4'-diaminodiphenylmethan (MOCA), 2,4- und 2,6-Toluylendiamin, Mischungen von 3,5-Dimethylthio-2,4- und -2,6-toluylendiamin (erhältlich als Ethacure® 300 von Albermarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink® von Air Products), 1,2-Bis(2-aminophenylthio)ethan, 2-Methylpropyl-(4-chloro-3,5-diaminobenzoat) oder tert.Butyl-(4-chloro-3,5-diaminobenzoat).
Der Härter kann weiterhin zusätzliche Addukte enthalten, insbesondere Addukte von 1,2-Propylendiamin mit aliphatischen Monoepoxiden oder mit Diepoxiden, oder Addukte von andern primären Diaminen mit Mono- oder Diepoxiden, sowie Umsetzungsprodukte aus Aminen und Epichlorhydrin, insbesondere jenes von 1,3-Bis(aminomethyl)benzol, kommerziell erhältlich als Gaskamine® 328 (von Mitsubishi Gas Chemical).
Der Härter kann weiterhin Polyamidoamine enthalten, welche Umsetzungsprodukte aus einer ein- oder mehrwertigen Carbonsäure, beziehungsweise deren Ester oder Anhydride, insbesondere einer Dimerfettsäure, und einem im stöchiometrischen Überschuss eingesetzten aliphatischen, cycloaliphatischen oder aromatischen Polyamin, insbesondere einem Polyalkylenamin wie beispielsweise DETA oder TETA, darstellen, insbesondere die kommerziell erhältlichen Polyamidoamine Versamid® 100, 125, 140 und 150 (von Cognis), Aradur® 223, 250 und 848 (von Huntsman), Euretek® 3607 und 530 (von Huntsman) und Beckopox® EH 651, EH 654, EH 655, EH 661 und EH 663 (von Cytec).
Der Härter kann weiterhin Mannich-Basen enthalten, welche Umsetzungsprodukte einer Mannich-Reaktion von Phenolen-mit Aldehyden, insbesondere Formaldehyd, und Polyaminen darstellen. Geeignet sind insbesondere die auch als Phenalkamine bezeichneten Mannich-Basen von Cardanol, beispielsweise die kommerziell erhältlichen Phenalkamine Cardolite® NC-541, NC-557, NC-558, NC-566, Lite 2001 und Lite 2002 (von Cardolite), Aradur® 3440, 3441, 3442 und 3460 (von Huntsman) und Beckopox® EH 614, EH 621, EH 624, EH 628 und EH 629 (von Cytec).
Der Härter kann weiterhin Monoamine wie Hexylamin und Benzylamin enthalten.
Der Härter kann weiterhin Mercaptogruppen aufweisende Verbindungen, enthalten, insbesondere die Folgenden:
- flüssige Mercaptan-terminierte Polysulfid-Polymere, bekannt unter dem Markennamen Thiokol® (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 und LP-2; sowie weiterhin bekannt unter dem Markennamen Thioplast® (von Akzo Nobel), insbesondere die Typen G 10, G 112, G 131, G 1, G 12, G 21, G 22, G 44 und G 4;
- Mercaptan-terminierte Polyoxyalkylen-Ether, erhältlich beispielsweise durch Umsetzung von Polyoxyalkylendi- und -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure® (von Cognis), insbesondere die Typen WR-8, LOF und 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat), sowie die Veresterungsprodukte von Polyoxyalkylendiolen und -triolen, ethoxyliertem Trimethylolpropan und Polyester-Diolen mit Thiocarbonsäuren wie Thioglykolsäure und 2- oder 3-Mercaptopropionsäure; und
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) und Ethandithiol.

Ein weiterer Gegenstand der Erfindung ist eine Epoxidharz-Zusammensetzung, enthaltend mindestens ein Epoxidharz und den vorgängig beschriebenen Härter.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.
Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf.
Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- und 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)methan, 2,2-Bis(4-hydroxy-3-methylyphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1-Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂-bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromo-neopentylglykol, Rizinusöl, Trimethylolpropan, Trimethylolethan, Pentaerythrol, Sorbit oder Glycerin, sowie alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, sowie Umsetzungsprodukte von Epichlorhydrin und Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Bevorzugt ist das Epoxidharz ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman und Momentive erhältlich sind. Diese Flüssigharze weisen eine für Epoxidharze niedrige Viskosität und im ausgehärteten Zustand gute Eigenschaften als Beschichtung auf. Sie können gegebenenfalls in Kombination mit Bisphenol A-Festharz oder Bisphenol-F-Novolak-Epoxidharz vorhanden sein.

Das Epoxidharz kann einen Reaktivverdünner, insbesondere einen mindestens eine Epoxidgruppe aufweisenden Reaktivverdünner, enthalten. Als Reaktivverdünner geeignet sind beispielsweise die Glycidylether von ein- oder mehrwertigen Phenolen oder aliphatischen oder cycloaliphatischen Alkoholen, wie insbesondere die bereits genannten Polyglycidylether von Di- oder Polyolen, sowie weiterhin insbesondere Phenylglycidylether, Kresylglycidylether, Benzylglycidylether, p-n-Butyl-phenylglycidylether, p-tert.Butyl-phenylglycidylether, Nonylphenylglycidylether, Allylglycidylether, Butylglycidylether, Hexylglycidylether, 2-Ethylhexylglycidylether, sowie Glycidylether von natürlichen Alkoholen, wie zum Beispiel C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, sowie eine Reduktion der Glasübergangstemperatur und der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten nicht einbaubaren Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate und Reaktivgruppen-aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere chlorsulfonierte Polyethylene und Fluor-haltige Polymere, Sulfonamid-modifizierte Melamine und gereinigte Montan-Wachse;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und/oder Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht und UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinol-diphosphat-Oligomer, Tetraphenylresorcinoldiphosphit, Ethylendiamindiphosphat oder Bisphenol-A-bis(diphenylphosphat), Tris(chloroethyl)phosphat, Tris(chloropropyl)phosphat und Tris(dichloroisopropyl)phosphat, Tris[3-bromo-2,2-bis(bromomethyl)propyl]phosphat, Tetrabromo-Bisphenol-A, Bis(2,3-dibromopropylether) von Bisphenol A, bromierte Epoxidharze, Ethylen-bis-(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine;
- oberflächenaktive Substanzen, insbesondere Netzmittel, Verlaufsmittel, Entlüftungsmittel und/oder Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und/oder Beschleuniger, insbesondere Salicylsäure und/oder 2,4,6-Tris(dimethylaminomethyl)phenol.
Bevorzugt enthält die Epoxidharz-Zusammensetzung keinen oder nur einen geringen Gehalt an nicht einbaubaren Verdünnern, besonders bevorzugt weniger als 10 Gewichts-%, insbesondere weniger als 5 Gewichts-%, am meisten bevorzugt weniger als 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.
Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Insbesondere ist die Epoxidharz-Zusammensetzung eine zweikomponentige Zusammensetzung, bestehend aus
(i) einer Harz-Komponente enthaltend mindestens ein Epoxidharz und
(ii) einer Härter-Komponente enthaltend den beschriebenen Härter.

Die Komponenten der zweikomponentigen Zusammensetzung werden jeweils in einem eigenen Gebinde gelagert. Weitere Bestandteile der zweikomponentigen Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der zweikomponentigen Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.
Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.
Mit der Vermischung der beiden Komponenten beginnt die Aushärtung durch chemische Reaktion, wie vorgängig beschrieben. Die Aushärtung erfolgt insbesondere bei Umgebungstemperatur. Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.
Ein weiterer Gegenstand der Erfindung ist somit auch eine ausgehärtete Zusammensetzung erhalten aus der Aushärtung einer Epoxidharz-Zusammensetzung wie im vorliegenden Dokument beschrieben.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips und Natursteine wie Granit oder Marmor;
- Metalle und Legierungen, wie Aluminium, Eisen, Stahl oder Buntmetalle, inklusive oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe oder weitere sogenannte Polymer-Composites;
- Kunststoffe, insbesondere Hart- und Weich-PVC, ABS, Polycarbonat (PC), Polyamid (PA), Polyester, PMMA, Epoxidharze, PUR, POM, PO, PE, PP, EPM oder EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) oder Sheet Moulding Compounds (SMC);
- beschichtete Substrate, wie pulverbeschichtete Metalle oder Legierungen;
- Farben oder Lacke.
Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Bestandteil von Faserverbundwerkstoffen (Composites), als Vergussmasse, Dichtstoff, Klebstoff, Belag, Beschichtung, Anstrich, Lack, Versiegelung, Grundierung oder Primer.
Insbesondere verwendbar ist sie als Vergussmasse, Dichtstoff und Klebstoff, wie beispielsweise als Elektrovergussmasse, Abdichtungsmasse, Karrosserieklebstoff, Sandwichelementklebstoff, Halbschalenklebstoff, beispielsweise für Rotorblätter von Windkraftanlagen, Brückenelementklebstoff oder Verankerungsklebstoff; sowie weiterhin als Belag, Beschichtung, Anstrich, Lack, Versiegelung, Grundierung und Primer für Bau- und Industrieanwendungen, wie insbesondere als Bodenbelag und Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden; sowie weiterhin als Voranstrich, Haftanstrich, Korrosionsschutz-Primer oder zur Hydrophobierung von Oberflächen. Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft verwendbar ist die beschriebene Epoxidharz-Zusammensetzung als Beschichtung. Als Beschichtung werden dabei flächig aufgebrachte Beläge aller Art verstanden, insbesondere auch Anstriche, Lacke, Versiegelungen, Grundierungen und Primer, wie vorgängig beschrieben. Insbesondere vorteilhaft verwendbar ist die beschriebene Epoxidharz-Zusammensetzung in emissionsarmen Produkten mit Öko-Gütesiegeln, beispielsweise nach Emicode (EC1 Plus), AgBB, DIBt, Der Blaue Engel, AFSSET, RTS (M1) und US Green Building Council (LEED).

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende Beschichtung auf überwiegend ebene Flächen oder als Anstrich appliziert werden kann. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 3'000 mPa·s, bevorzugt im Bereich von 300 bis 2'000 mPa·s, besonders bevorzugt im Bereich von 300 bis 1'500 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann aber auch mit einem Pinsel oder Roller oder als Spritzapplikation erfolgen, beispielsweise als Korrosionsschutzbeschichtung auf Stahl.
Bei der Aushärtung entstehen typischerweise weitgehend klare, glänzende und nichtklebrige Filme von hoher Härte, welche eine gute Haftung zu verschiedensten Substraten aufweisen.

Ein weiterer Gegenstand der Erfindung ist ein Artikel umfassend eine ausgehärtete Zusammensetzung, erhalten durch die Aushärtung der beschriebenen Epoxidharz-Zusammensetzung. Die ausgehärtete Zusammensetzung liegt dabei insbesondere in Form einer Beschichtung vor.

Die beschriebene Epoxidharz-Zusammensetzung zeichnet sich durch vorteilhafte Eigenschaften aus. Sie ist sehr niedrigviskos und geruchsarm und härtet auch bei feucht-kaltenBedingungen schnell und weitgehend ohne Blushing-Effekte aus, sogar mit geringen Anteilen oder ganz ohne die Verwendung von nicht einbaubaren Verdünnern, und insbesondere auch ohne die Verwendung von leichtflüchtigen, geruchsintensiven Aminen. Bei der flächigen Verwendung entstehen klare, nichtklebrige Filme von hoher Härte und hoher Oberflächenqualität, welche unter Lichteinfluss kaum vergilben. Mit der beschriebenen Epoxidharz-Zusammensetzung sind insbesondere emissionsarme Epoxidharz-Produkte zugänglich, welche die Bedingungen für viele Öko-Gütesiegel erfüllen und gleichzeitig hohen Ansprüchen bezüglich Arbeitssicherheit, Verarbeitungs- und Gebrauchseigenschaften genügen.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.
"AHEW" steht für das Aminwasserstoff-Equivalentgewicht.
"EEW" steht für das Epoxid-Equivalentgewicht.

### 1. Beschreibung der Messmethoden

**Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).

Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.
Die **Aminzahl** wurde durch Titration bestimmt (mit 0.1N HClO₄ in Essigsäure gegen Kristallviolett).
Der **Schmelzpunkt** wurde bestimmt mittels DMTA-Messung im Temperaturbereich von -50 °C bis 25 °C, bei 5 K/min und 10 Hz.

### 2. Verwendete kommerzielle Substanzen:

| | |
|---|---|
| Araldite® DY-K: | (von Huntsman), 2-Kresylglycidylether, EEW ca. 183 g/Eq |
| Araldite® DY-P: | (von Huntsman), p-tert.Butylphenylglycidylether, EEW ca. 225 g/Eq |
| Cardolite® LITE | (von Cardolite), Glycidylether von Cardanol, EEW ca. 415 |
| 2513HP: | g/Eq |
| Araldite® GY 250: | (von Huntsman), Bisphenol-A-Diglycidylether,EEW ca. 187.5 g/Eq |
| Araldite® DY-E: | (von Huntsman), Monoglycidylether von C₁₂-bis C₁₄-Alkoholen, EEW ca. 290 g/Eq |
| Ancamine® K 54: | (von Air Products), 2,4,6-Tris(dimethylaminomethyl)phenol |
| Dytek® A: | (von Invista),1,5-Diamino-2-methylpentan, AHEW 29.0 g/Eq |
| MXDA: | (von Mitsubishi Gas Chemical), 1,3-Bis(aminomethyl)benzol |
| Gaskamine® 240: | (von Mitsubishi Gas Chemical), styrolisiertes 1,3-Bis(aminomethyl)benzol, AHEW ca. 103 g/Eq |
| Jeffamine® D-230: | (von Huntsman), Polyoxypropylendiamin mit mittlerem Molekulargewicht ca. 240 g/mol, AHEW 60 g/Eq |
| Jeffamine® RFD- | (von Huntsman), cycloaliphatisches ethergruppenhaltiges |
| 270: | Diamin aus der Propoxylierung und nachfolgenden Aminierung von 1,4-Dimethylolcyclohexan, mittleres Molekulargewicht ca. 270 g/mol, AHEW 67 g/Eq |
| Vestamin® TMD: | (von Evonik), 2,2,4- und 2,4,4-Trimethylhexamethylendiamin, AHEW 39.6 g/Eq |
| 1,3-BAC: | (von Mitsubishi Gas Chemical), 1,3-Bis(aminomethyl)cyclohexan, AHEW 35.5 g/Eq |

### 3. Herstellung von Aminen:

**Amin A-1:** 1-((2-Aminopropyl)amino)-3-(2-methylphenoxy)propan-2-ol 4.15 kg (56 mol) 1,2-Propylendiamin wurden unter Stickstoffatmosphäre vorgelegt, auf 70 °C aufgewärmt und unter gutem Rühren langsam mit 2.93 kg (16 mol) Araldite® DY-K versetzt, wobei die Temperatur der Reaktionsmischung durch Kühlung zwischen 70 und 80 °C gehalten wurde. Die Reaktionsmischung wurde während 1 Stunde bei 80°C belassen, anschliessend abgekühlt und die flüchtigen Bestandteile mittels Dünnschichtverdampfer (0.5-1 mbar, Manteltemperatur 115 °C) entfernt. Es wurde eine klare, leicht gelbliche Flüssigkeit mit einer Viskosität bei 20 °C von 3.3 Pa·s, einer Aminzahl von 478.7 mg KOH/g, einer Reinheit von 91.5% (bestimmt mittels Gas-Chromatographie, 8.5% 1,3-Bis(2-methylphenoxy)propan-2-ol aus Araldite® DY-K), einem Schmelzpunkt von -25 °C und einem theoretischen AHEW von ca. 85.7 g/Eq erhalten.
FT-IR: 3025, 2955, 2918, 1601, 1590, 1494, 1456, 1377, 1307, 1288, 1242, 1191, 1120, 1050, 1035, 926, 837, 748, 713.

**Amin A-2:** 1-((2-Aminopropyl)amino)-3-(4-tert.butylphenoxy)propan-2-ol 77.4 g (1.04 mol) 1,2-Propylendiamin wurden unter Stickstoffatmosphäre vorgelegt, auf 70 °C aufgewärmt und unter gutem Rühren langsam mit 67.5 g (0.3 mol) Araldite® DY-P versetzt. Die Reaktionsmischung wurde während 2 Stunden bei 80°C belassen. Anschliessend wurden die flüchtigen Bestandteile am Rotationsverdampfer bei 65 °C und 1 mbar während 3 Stunden entfernt. Es wurde eine klare, leicht gelbliche Flüssigkeit mit einer Viskosität von 105 Pa·s, einer Aminzahl von 374.9 mg KOH/g und einem theoretischen AHEW von ca. 99.7 g/Eq erhalten.

### Amin A-3:

Wie für Amin **A-2** beschrieben wurden 51.9 g (0.7 mol) 1,2-Propylendiamin mit 83.0 g (0.2 mol) Cardolite® LITE 2513HP umgesetzt. Es wurde eine klare, leicht gelbliche Flüssigkeit mit einer Viskosität von 1.93 Pa·s, einer Aminzahl von 204.3 mg KOH/g und einem theoretischen AHEW von ca. 163.0 g/Eq erhalten.

### Amin A-4: (Vergleich)

Wie für Amin **A-1** beschrieben wurden 4.65 kg (40 mol) Dytek® A mit 1.83 kg (10 mol) Araldite® DY-K umgesetzt, wobei die Manteltemperatur des Dünnschichtverdampfers 160 °C betrug. Es wurde eine klare, leicht gelbliche Flüssigkeit mit einer Viskosität bei 20 °C von 6.5 Pa·s, einer Aminzahl von 367.1 mg KOH/g und einem theoretischen AHEW von ca. 99.7 g/Eq erhalten.

### Amin A-5: (Vergleich)

Wie für Amin **A-2** beschrieben wurden 96.2 g (1.6 mol) Ethylendiamin mit 73.2 g (0.4 mol) Araldite® DY-K umgesetzt. Es wurde eine klare, leicht gelbliche Flüssigkeit mit einer Viskosität von 8.26 Pa·s, einer Aminzahl von 484.9 mg KOH/g und einem theoretischen AHEW von ca. 81.0 g/Eq erhalten.

Beim den Aminen **A-1** bis **A-3** handelt es sich um Amine der Formel (I); die Amine **A-4** und **A-5** dienen als Vergleich.

### 4. Herstellung von alkylierten Aminen

### benzyliertes MXDA:

In einem Rundkolben wurden 17.0 g (0.16 mol) Benzaldehyd und 13.6 g (0.10 mol) MXDA unter Stickstoffatmosphäre in ausreichend Isopropanol gelöst. Die Lösung wurde während 30 Minuten bei 23°C gerührt und anschliessend bei einem Wasserstoff-Druck von 80 bar, einer Temperatur von 80°C und einem Fluss von 3 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die Lösung im Vakuum bei 80°C eingeengt. Erhalten wurde ein klares, gelbliches Öl mit einer Viskosität von 0.1 Pa·s bei 20°C, einer Aminzahl von 416.8 mg KOH/g und einem theoretischen AHEW von ca. 115.5 g/Eq.

### ethylhexyliertes MXDA:

Auf die gleiche Weise wie für das benzylierte MXDA beschrieben wurden 25.6 g (0.20 mol) 2-Ethylhexanal und 13.6 g (0.10 mol) MXDA umgesetzt. Erhalten wurde eine klare, leicht gelbliche Flüssigkeit mit einer Viskosität von 140 mPa·s bei 20 °C, einer Aminzahl von 308.6 mg KOH/g und einem theoretischen AHEW von ca. 180.3 g/Eq.

### 5. Herstellung von Härtern und Epoxidharz-Zusammensetzungen

Für jedes Beispiel wurden die in den Tabellen 1 bis 4 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.

Ebenso wurden die in den Tabellen 1 bis 4 angegebenen Inhaltsstoffe der Harz-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C bestimmt ("**Viskosität (10')**")**.**

Ein erster Film wurde in einer Schichtdicke von 500 µm auf eine Glasplatte aufgezogen und dieser bei 23±1 °C und 50±5 % relativer Feuchtigkeit (= Normklima, im folgenden abgekürzt mit "NK") gelagert, beziehungsweise ausgehärtet. An diesem Film wurde die **Königshärte** (Pendelhärte nach König, gemessen nach DIN EN ISO 1522) nach 2 Tagen ("Königshärte (NK) (2d)"), nach 4 Tagen ("Königshärte (NK) (4d)"), nach 7 Tagen ("Königshärte (NK) (7d)") und nach 14 Tagen ("Königshärte (NK) (14d)") bestimmt. Nach 14 Tagen wurde der Aspekt des Films beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.

Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 3 Wochen im NK gelagert, beziehungsweise ausgehärtet. 24 Stunden nach der Applikation wurde ein Flaschendeckel aus Polypropylen auf den Film aufgesetzt, unter welchem ein feuchtes Schwämmchen platziert war. Nach weiteren 24 Stunden wurde das Schwämmchen und der Deckel entfernt und an einer neuen Stelle des Films platziert, wo es nach 24 Stunden wieder entfernt und neu platziert wurde, insgesamt 4 mal. Anschliessend wurde der Aspekt dieses Films beurteilt (in den Tabellen mit "Aspekt (8°/80%)" bezeichnet), auf die gleiche Weise wie für den Aspekt (NK) beschrieben. Dabei wurde jeweils auch die Anzahl Markierungen angegeben, die im Film durch das feuchte Schwämmchen und/ oder den aufgesetzten Deckel sichtbar waren. An den so ausgehärteten Filmen wurde wiederum die Königshärte bestimmt, jeweils nach 7 Tagen bei 8 °C und 80 % relativer Feuchtigkeit ("Königsh. (7d 8°/80%)"), dann nach weiteren 2 Tagen im NK ("Königsh. (+2d NK)"), 7 Tagen im NK ("Königsh. (+7d NK)") und nach 14 Tagen im NK ("Königsh. (+14d NK)").

Die Resultate sind in den Tabellen 1 bis 4 angegeben.

Bei den Epoxidharz-Zusammensetzungen ***EZ-1*** bis ***EZ-16*** handelt es sich um erfindungsgemässe Beispiele. Bei den Epoxidharz-Zusammensetzungen ***Ref-1*** bis ***Ref-9*** handelt es sich um Vergleichsbeispiele.

**Tabelle 1: Zusammensetzung und Eigenschaften von EZ-1 bis EZ-4 und Ref-1 bis Ref-3. "n.m." steht für "nicht messbar". "n.b." steht für "nicht bestimmt".**

| **Beispiel** | | | ***EZ-1*** | ***EZ-2*** | ***EZ-3*** | ***EZ-4*** | ***Ref-1*** | ***Ref-2*** | ***Ref-3*** |
|---|---|---|---|---|---|---|---|---|---|
| **Harz-Komp.:** | | | | | | | | | |
| | Araldite® GY-250 | | 167.2 | 167.2 | 167.2 | 167.2 | 167.2 | 167.2 | 167.2 |
| | Araldite® DY-E | | 31.8 | 31.8 | 31.8 | 31.8 | 31.8 | 31.8 | 31.8 |

| **Härter-Komp.:** | | | | | | | | | |
|---|---|---|---|---|---|---|---|---|---|
| | Amin | | **A-1** | **A-1** | **A-2** | **A-3** | **A-4** | **A-4** | **A-5** |
| | | | 85.7 | 85.7 | 99.7 | 163.0 | 99.7 | 99.7 | 81.0 |
| | Ancamine® K 54 | | - | 2.8 | 2.9 | 2.9 | - | 2.9 | 2.8 |
| | Viskosität (10') [Pa·s] | | 2.19 | 2.24 | 6.05 | 1.81 | 3.49 | 3.63 | 8.51 |
| Königshärte [s] | | (1d NK) | 105 | 137 | 119 | n.m. | 64 | 101 | 151 |
| | | (2d NK) | 165 | 186 | 175 | 9 | 98 | 139 | 176 |
| | | (4d NK) | 199 | 210 | 204 | 18 | 125 | 166 | 195 |
| | | (7d NK) | 214 | 221 | 220 | 27 | 148 | 182 | 200 |
| | | (14d NK) | 218 | 220 | 221 | 38 | 171 | 180 | 204 |
| Aspekt (NK) | | | schön | schön | schön | schön | schön | schön | milchig |
| Königsh. [s] | | (7d 8°/80%) | 59 | 81 | 83 | n.m. | 35 | 52 | 87 |
| | | (+2d NK) | 175 | 183 | 181 | n.m. | 88 | 126 | 172 |
| | | (+7d NK) | 206 | 198 | 221 | n.m. | 125 | 168 | 196 |
| | | (+14d NK) | 205 | 203 | 220 | n.m. | 151 | 175 | 198 |
| Aspekt (8°/80%) Anzahl Markierungen | | | schön 1 | schön 1 | schön 2 | klebrig n.b. | schön 1 | schön 1 | milchig 3 |

**Tabelle 2: Zusammensetzung und Eigenschaften von EZ-5 bis EZ-11. "l." steht für "leicht"**

| **Beispiel** | | | ***EZ-5*** | ***EZ-6*** | ***EZ-7*** | ***EZ-8*** | ***EZ-9*** | ***EZ-10*** | ***EZ-11*** |
|---|---|---|---|---|---|---|---|---|---|
| **Harz-Komp.:** | | | | | | | | | |
| | Araldite® GY-250 | | 167.2 | 167.2 | 167.2 | 167.2 | 167.2 | 167.2 | 167.2 |
| | Araldite® DY-E | | 31.8 | 31.8 | 31.8 | 31.8 | 31.8 | 31.8 | 31.8 |

| **Härter-Komp.:** | | | | | | | | | |
|---|---|---|---|---|---|---|---|---|---|
| | Amin | | **A-1** | **A-1** | **A-1** | **A-1** | **A-1** | **A-1** | **A-2** |
| | | | 51.4 | 34.3 | 42.9 | 42.9 | 42.9 | 42.9 | 59.8 |
| | Jeffamine® D-230 | | 24.0 | 24.0 | - | 18.0 | 21.0 | 21.0 | 24.0 |
| | Jeffamine® RFD-270 | | - | - | 20.1 | - | - | - | - |
| | Gaskamine® 240 | | - | 20.6 | - | - | - | - | - |
| | ethylhexyliertes MXDA | | - | - | 36.0 | - | - | - | - |
| | benzyliertes MXDA | | - | - | - | 23.1 | - | - | - |
| | 1,3-BAC | | - | - | - | - | 5.3 | - | - |
| | Vestamin®TMD | | - | - | - | - | - | 5.9 | - |
| | Ancamine® K 54 | | 2.7 | 2.8 | 3.0 | 2.8 | 2.7 | 2.7 | 2.8 |
| Viskosität (10') [Pa·s] | | | 1.07 | 0.79 | 0.56 | 0.91 | 0.97 | 0.94 | 1.73 |
| Königshärte [s] | | (1d NK) | 74 | 75 | 33 | 74 | 84 | 46 | 60 |
| | | (2d NK) | 160 | 148 | 81 | 153 | 122 | 104 | 143 |
| | | (4d NK) | 185 | 186 | 110 | 190 | 170 | 181 | 198 |
| | | (7d NK) | 206 | 200 | 133 | 203 | 171 | 206 | 219 |
| | | (14d NK) | 207 | 218 | 143 | 213 | 179 | 214 | 218 |
| Aspekt (NK) | | | schön | schön | schön | schön | I. Struktur | schön | schön |
| Königsh. [s] | | (7d 8°/80%) | 50 | 47 | 25 | 47 | 33 | 24 | 53 |
| | | (+2d NK) | 151 | 160 | 77 | 167 | 104 | 81 | 165 |
| | | (+7d NK) | 165 | 206 | 111 | 203 | 151 | 116 | 193 |
| | | (+14d NK) | 168 | 205 | 115 | 204 | 153 | 121 | 198 |
| Aspekt (8°/80%) Anzahl Markierungen | | | schön 1 | schön 1 | schön keine | schön 1 | schön 2 | schön 2 | schön 2 |

**Tabelle 3: Zusammensetzung und Eigenschaften von Ref-4 bis Ref-9. "l." steht für "leicht"**

| **Beispiel** | | | ***Ref-4*** | ***Ref-5*** | ***Ref-6*** | ***Ref-7*** | ***Ref-8*** | ***Ref-9*** |
|---|---|---|---|---|---|---|---|---|
| **Harz-Komp.:** | | | | | | | | |
| | Araldite® GY-250 | | 167.2 | 167.2 | 167.2 | 167.2 | 167.2 | 167.2 |
| | Araldite® DY-E | | 31.8 | 31.8 | 31.8 | 31.8 | 31.8 | 31.8 |

| **Härter-Komp.:** | | | | | | | | |
|---|---|---|---|---|---|---|---|---|
| | Amin | | **A-4** | **A-4** | **A-4** | **A-5** | **A-5** | **A-5** |
| | | | 59.8 | 39.9 | 49.9 | 48.6 | 32.4 | 40.5 |
| | Jeffamine® D-230 | | 24.0 | 24.0 | 21.0 | 24.0 | 24.0 | 21.0 |
| Gaskamine® 240 | | | - | 20.6 | - | - | 20.6 | - |
| | 1,3-BAC | | - | - | 5.3 | - | - | 5.3 |
| Ancamine® K 54 | | | 2.8 | 2.8 | 2.7 | 2.7 | 2.8 | 2.7 |
| Viskosität (10') [Pa·s] | | | 1.21 | 0.93 | 1.13 | 2.13 | 0.97 | 1.24 |
| Königshärte [s] | | (1d NK) | 50 | 42 | 75 | 89 | 63 | 108 |
| | | (2d NK) | 112 | 102 | 148 | 141 | 123 | 158 |
| | | (4d NK) | 155 | 155 | 186 | 181 | 166 | 190 |
| | | (7d NK) | 183 | 173 | 195 | 191 | 183 | 202 |
| | | (14d NK) | 200 | 192 | 203 | 204 | 197 | 213 |
| Aspekt (NK) | | | schön | schön | schön | l. milchig | schön | schön |
| Königsh. [s] | | (7d 8°/80%) | 35 | 25 | 26 | 47 | 32 | 29 |
| | | (+2d NK) | 132 | 111 | 46 | 151 | 109 | 91 |
| | | (+7d NK) | 175 | 169 | 77 | 182 | 160 | 119 |
| | | (+14d NK) | 188 | 176 | 79 | 185 | 164 | 122 |
| Aspekt (8°/80%) | | | schön | schön | l. klebrig | l. milchig | schön | schön |
| Anzahl Markierungen | | | 1 | 1 | 2 | 1 | 1 | 2 |

**Tabelle 4: Zusammensetzung und Eigenschaften von EZ-12 bis EZ-16. ¹ gelöst in den vorher vermischten Aminen**

| **Beispiel** | | | ***EZ-12*** | ***EZ-13*** | ***EZ-14*** | ***EZ-15*** | ***EZ-16*** |
|---|---|---|---|---|---|---|---|
| **Harz-Komp.:** | | | | | | | |
| | Araldite® GY-250 | | 167.2 | 167.2 | 167.2 | 167.2 | 167.2 |
| | Araldite® DY-E | | 31.8 | 31.8 | 31.8 | 31.8 | 31.8 |

| **Härter-Komp.:** | | | | | | | |
|---|---|---|---|---|---|---|---|
| | Amin | | **A-1** | **A-1** | **A-1** | **A-1** | **A-1** |
| | | | 79.5 | 89.9 | 47.7 | 54.0 | 31.8 |
| | Jeffamine® D-230 | | - | - | 24.0 | 24.0 | 24.0 |
| | Gaskamine® 240 | | - | - | - | - | 20.6 |
| | Salicylsäure¹ | | 1.6 | 4.8 | 1.0 | 2.9 | 1.6 |
| | Ancamine® K 54 | | 0.8 | 1.9 | 0.7 | 1.2 | 0.8 |
| Viskosität (10') [Pa·s] | | | 2.86 | 11.10 | 1.16 | 2.40 | 0.92 |
| Königshärte [s] | | (1d NK) | 115 | 83 | 56 | 45 | 43 |
| | | (2d NK) | 171 | 150 | 146 | 126 | 111 |
| | | (4d NK) | 200 | 185 | 188 | 178 | 169 |
| | | (7d NK) | 219 | 197 | 209 | 197 | 189 |
| | | (14d NK) | 219 | 217 | 211 | 213 | 201 |
| Aspekt (NK) | | | schön | schön | schön | schön | schön |
| Königsh. [s] | | (7d 8°/80%) | 68 | 59 | 42 | 36 | 36 |
| | | (+2d NK) | 175 | 171 | 161 | 148 | 148 |
| | | (+7d NK) | 219 | 199 | 185 | 192 | 175 |
| | | (+14d NK) | 216 | 218 | 203 | 207 | 188 |
| Aspekt (8°/80%) | | | schön | schön | schön | schön | schön |
| Anzahl Markierungen | | | 1 | 1 | 1 | keine | 1 |

## Patentansprüche

1. Amin der Formel (I), 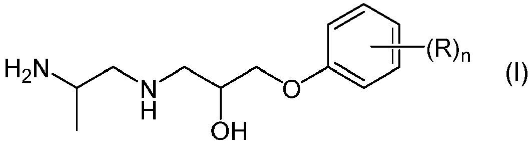 wobei
R für einen Alkyl-, Cycloalkyl-, Aralkyl- oder Alkoxy-Rest mit 1 bis 22 Kohlenstoffatomen, welcher gegebenenfalls ungesättigte Anteile enthält, steht; und
n für 0 oder 1 oder 2 oder 3 steht.

2. Amin gemäss Anspruch 1, **dadurch gekennzeichnet, dass** n für 1 steht.

3. Amin gemäss einem der Ansprüche 1 oder 2, **dadurch gekennzeichnet, dass** R für Methyl steht.

4. Verfahren zur Herstellung eines Amins gemäss einem der Ansprüche 1 bis 3 durch Umsetzung von 1,2-Propylendiamin mit einem Arylglycidylether im Molverhältnis 1,2-Propylendiamin/Arylglycidylether im Bereich von 1.1 bis 5 und nachfolgender destillativer Entfernung von unreagiertem 1,2-Propylendiamin.

5. Verwendung mindestens eines Amins gemäss einem der Ansprüche 1 bis 3 in einem Härter für Epoxidharze.

6. Verwendung gemäss Anspruch 5, **dadurch gekennzeichnet, dass** der Härter ein Amin der Formel (I) in einer solchen Menge enthält, dass 5 bis 100% der gegenüber Epoxidgruppen reaktiven Aminwasserstoffe im Härter vom Amin der Formel (I) stammen.

7. Verwendung gemäss einem der Ansprüche 5 oder 6, **dadurch gekennzeichnet, dass** der Härter neben dem Amin der Formel (I) mindestens ein weiteres Polyamin mit mindestens zwei gegenüber Epoxidgruppen aktiven Aminwasserstoffen enthält.

8. Verwendung gemäss Anspruch 7, **dadurch gekennzeichnet, dass** das weitere Polyamin ein primäres Diamin ausgewählt aus der Gruppe bestehend aus 2,2,4- und 2,4,4-Trimethylhexamethylendiamin, 1-Amino-3-aminomethyl-3,5,5-trimethylcyclohexan, 1,3-Bis(aminomethyl)benzol, 1,3-Bis(aminomethyl)cyclohexan, 1,4-Bis(aminomethyl)cyclohexan, Bis(4-aminocyclohexyl)methan, Bis(4-amino-3-methylcyclohexyl)methan, 2,5(2,6)-Bis(aminomethyl)bicyclo[2.2.1]heptan, 3(4),8(9)-Bis(aminomethyl)tricyclo[5.2.1.0^{2,6}]decan und Ethergruppen-haltigen Polyaminen mit einem mittleren Molekulargewicht bis zu 500 g/mol ist.

9. Verwendung gemäss Anspruch 7, **dadurch gekennzeichnet, dass** das weitere Polyamin ein Polyamin mit mindestens einer sekundären Aminogruppe ausgewählt aus der Gruppe bestehend aus N-monoalkyliertem 1,6-Hexandiamin, N,N'-dialkyliertem 1,6-Hexandiamin, N-monoalkyliertem 1,5-Diamino-2-methylpentan, N,N'-dialkyliertem 1,5-Diamino-2-methylpentan, N-monoalkyliertem 1,3-Bis(aminomethyl)cyclohexan, N,N'-dialkyliertem 1,3-Bis(aminomethyl)cyclohexan, N-monoalkyliertem 1,4-Bis(aminomethyl)cyclohexan, N,N'-dialkyliertem 1,4-Bis(aminomethyl)cyclohexan, N-monoalkyliertem 1,3-Bis(aminomethyl)benzol, N,N'-dialkyliertem 1,3-Bis(aminomethyl)benzol, N-monoalkyliertem BHMT, N,N'-dialkyliertem BHMT, N-monoalkyliertem DETA, N,N'-dialkyliertem DETA, N-monoalkyliertem TETA, N,N'-dialkyliertem TETA, N-monoalkyliertem TEPA, N,N'-dialkyliertem TEPA, N-monoalkyliertem DPTA, N,N'-dialkyliertem DPTA, N-monoalkyliertem N3-Amin, N,N'-dialkyliertem N3-Amin, N-monoalkyliertem N4-Amin und N,N'-dialkyliertem N4-Amin, wobei die Alkylgruppen jeweils ausgewählt sind aus der Gruppe bestehend aus Benzyl, 2-Phenylethyl, Isobutyl, Hexyl und 2-Ethylhexyl.

10. Verwendung gemäss einem der Ansprüche 7 bis 9, **dadurch gekennzeichnet, dass** das weitere Polyamin ein Ethergruppen aufweisendes Diamin ist.

11. Verwendung gemäss einem der Ansprüche 5 bis 10, **dadurch gekennzeichnet, dass** der Härter einen Gehalt an nicht einbaubaren Verdünnern von weniger als 25 Gewichts-% aufweist.

12. Epoxidharz-Zusammensetzung enthaltend mindestens ein Epoxidharz und einen Härter, wie er in einem der Ansprüche 5 bis 11 beschrieben ist.

13. Epoxidharz-Zusammensetzung gemäss Anspruch 12, **dadurch gekennzeichnet, dass** sie eine zweikomponentige Zusammensetzung ist, bestehend aus
(i) einer Harz-Komponente enthaltend mindestens ein Epoxidharz und
(ii) einer Härter-Komponente enthaltend den Härter.

14. Ausgehärtete Zusammensetzung erhalten aus der Aushärtung einer Epoxidharz-Zusammensetzung gemäss einem der Ansprüche 12 oder 13.

15. Artikel umfassend eine ausgehärtete Zusammensetzung gemäss Anspruch 14.

## Claims

1. An amine of the formula (I), 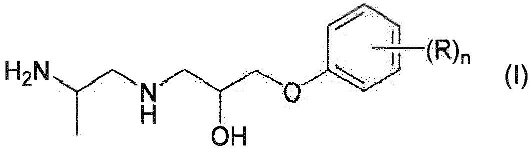 where
R is an alkyl, cycloalkyl, aralkyl or alkoxy radical having 1 to 22 carbon atoms which optionally comprises unsaturated fractions; and
n is 0 or 1 or 2 or 3.

2. The amine as claimed in claim 1, **characterized in that** n is 1.

3. The amine as claimed in either of claims 1 and 2, **characterized in that** R is methyl.

4. A process for preparing an amine as claimed in any of claims 1 to 3 by reacting 1,2-propylenediamine with an aryl glycidyl ether in a 1,2-propylenediamine/aryl glycidyl ether molar ratio in the range from 1.1 to 5 and subsequent distillative removal of unreacted 1,2-propylenediamine.

5. The use of at least one amine as claimed in any of claims 1 to 3 in a hardener for epoxy resins.

6. The use as claimed in claim 5, **characterized in that** the hardener comprises an amine of formula (I) in an amount such that 5 to 100% of the amine hydrogens in the hardener that are reactive toward epoxide groups originate from the amine of the formula (I).

7. The use as claimed in either of claims 5 and 6, **characterized in that** the hardener as well as the amine of the formula (I) comprises at least one further polyamine having at least two amine hydrogens that are active toward epoxide groups.

8. The use as claimed in claim 7, **characterized in that** the further polyamine is a primary diamine selected from the group consisting of 2,2,4- and 2,4,4-trimethylhexamethylenediamine, 1-amino-3-aminomethyl-3,5,5-trimethylcyclohexane, 1,3-bis(aminomethyl)benzene, 1,3-bis(aminomethyl)cyclohexane, 1,4-bis(aminomethyl)cyclohexane, bis(4-aminocyclohexyl)methane, bis(4-amino-3-methylcyclohexyl)methane, 2,5(2,6)-bis(aminomethyl)bicyclo[2.2.1]heptane, 3(4),8(9)-bis(aminomethyl)tricyclo[5.2.1.0^{2,6}]decane, and polyamines containing ether groups and having an average molecular weight of up to 500 g/mol.

9. The use as claimed in claim 7, **characterized in that** the further polyamine is a polyamine having at least one secondary amino group, selected from the group consisting of N-monoalkylated 1,6-hexanediamine, N,N'-dialkylated 1,6-hexanediamine, N-monoalkylated 1,5-diamino-2-methylpentane, N,N'-dialkylated 1,5-diamino-2-methylpentane, N-monoalkylated 1,3-bis(aminomethyl)cyclohexane, N,N'-dialkylated 1,3-bis(aminomethyl)cyclohexane, N-monoalkylated 1,4-bis(aminomethyl)cyclohexane, N,N'-dialkylated 1,4-bis(aminomethyl)cyclohexane, N-monoalkylated 1,3-bis(aminomethyl)benzene, N,N'-dialkylated 1,3-bis(aminomethyl)benzene, N-monoalkylated BHMT, N,N'-dialkylated BHMT, N-monoalkylated DETA, N,N'-dialkylated DETA, N-monoalkylated TETA, N,N'-dialkylated TETA, N-monoalkylated TEPA, N,N'-dialkylated TEPA, N-monoalkylated DPTA, N,N'-dialkylated DPTA, N-monoalkylated N3-amine, N,N'-dialkylated N3-amine, N-monoalkylated N4-amine and N,N'-dialkylated N4-amine, the alkyl groups being selected in each case from the group consisting of benzyl, 2-phenylethyl, isobutyl, hexyl, and 2-ethylhexyl.

10. The use as claimed in any of claims 7 to 9, **characterized in that** the further polyamine is a diamine containing ether groups.

11. The use as claimed in any of claims 5 to 10, **characterized in that** the hardener contains less than 25 weight% of unincorporable diluents.

12. An epoxy resin composition comprising at least one epoxy resin and a hardener as described in any of claims 5 to 11.

13. The epoxy resin composition as claimed in claim 12, **characterized in that** it is a two-pack composition consisting of
(i) a resin component comprising at least one epoxy resin, and
(ii) a hardener component comprising the hardener.

14. A cured composition obtained from the curing of an epoxy resin composition as claimed in either of claims 12 and 13.

15. An article comprising a cured composition as claimed in claim 14.

## Revendications

1. Amine de formule (I) 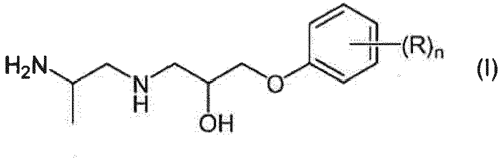 dans laquelle
R représente un radical alkyle, cycloalkyle, aralkyle ou alcoxy de 1 à 22 atomes de carbone, qui contient éventuellement des fractions insaturées ; et
n représente 0 ou 1 ou 2 ou 3.

2. Amine selon la revendication 1, **caractérisée en ce que** n représente 1.

3. Amine selon l'une quelconque des revendications 1 ou 2, **caractérisée en ce que** R représente méthyle.

4. Procédé de fabrication d'une amine selon l'une quelconque des revendications 1 à 3 par mise en réaction de 1,2-propylène-diamine avec un éther arylglycidylique en un rapport molaire 1,2-propylène-diamine/éther arylglycidylique dans la plage allant de 1,1 à 5, puis élimination par distillation de la 1,2-propylène-diamine non réagie.

5. Utilisation d'au moins une amine selon l'une quelconque des revendications 1 à 3 dans un durcisseur pour résines époxyde.

6. Utilisation selon la revendication 5, **caractérisée en ce que** le durcisseur contient une amine de formule (I) en une quantité telle que 5 à 100 % des hydrogènes d'amine réactifs avec les groupes époxyde dans le durcisseur proviennent de l'amine de formule (I).

7. Utilisation selon l'une quelconque des revendications 5 ou 6, **caractérisée en ce que** le durcisseur contient en plus de l'amine de formule (1) au moins une polyamine supplémentaire contenant au moins deux hydrogènes d'amine actifs envers les groupes époxyde.

8. Utilisation selon la revendication 7, **caractérisée en ce que** la polyamine supplémentaire est une diamine primaire choisie dans le groupe constitué par la 2,2,4-et 2,4,4-triméthylhexaméthylène-diamine, le 1-amino-3-aminométhyl-3,5,5-triméthylcyclohexane, le 1,3-bis(aminométhyl)benzène, le 1,3-bis(aminométhyl)cyclohexane, le 1,4-bis(aminométhyl)cyclohexane, le bis(4-aminocyclohexyl)méthane, le bis(4-amino-3-méthylcyclohexyl)méthane, le 2,5(2,6)-bis(aminométhyl)bicyclo[2.2.1]heptane, le 3(4),8(9)-bis (aminométhyl) tricycle [5.2.1.0^{2,6}] décane et les polyamines contenant des groupes éther ayant un poids moléculaire moyen de jusqu'à 500 g/mol.

9. Utilisation selon la revendication 7, **caractérisée en ce que** la polyamine supplémentaire est une polyamine contenant au moins un groupe amino secondaire choisi dans le groupe constitué par la 1,6-hexane-diamine N-monoalkylée, la 1,6-hexane-diamine N,N'-dialkylée, le 1,5-diamino-2-méthylpentane N-monoalkylé, le 1,5-diamino-2-méthyl-pentane N,N'-dialkylé, le 1,3-bis(aminométhyl)cyclohexane N-monoalkylé, le 1,3-bis(aminométhyl)cyclohexane N,N'-dialkylé, le 1,4-bis(aminométhyl)cyclohexane N-monoalkylé, le 1,4-bis(aminométhyl)cyclohexane N,N'-dialkylé, le 1,3-bis(aminométhyl)benzène N-monoalkylé, le 1,3-bis(aminométhyl)benzène N,N'-dialkylé, la BHMT N-monoalkylée, la BHMT N,N'-dialkylée, la DETA N-monoalkylée, la DETA N,N'-dialkylée, la TETA N-monoalkylée, la TETA N,N'-dialkylée, la TEPA N-monoalkylée, la TEPA N,N'-dialkylée, la DPTA N-monoalkylée, la DPTA N,N'-dialkylée, la N3-amine N-monoalkylée, la N3-amine N,N'-dialkylée, la N4-amine N-monoalkylée et la N4-amine N,N'-dialkylée, les groupes alkyle étant à chaque fois choisis dans le groupe constitué par benzyle, 2-phényléthyle, isobutyle, hexyle et 2-éthylhexyle.

10. Utilisation selon l'une quelconque des revendications 7 à 9, **caractérisée en ce que** la polyamine supplémentaire est une diamine comprenant des groupes éther.

11. Utilisation selon l'une quelconque des revendications 5 à 10, **caractérisée en ce que** le durcisseur présente une teneur en diluant non incorporable de moins de 25 % en poids.

12. Composition de résine époxyde contenant au moins une résine époxyde et un durcisseur, tel que décrit dans l'une quelconque des revendications 5 à 11.

13. Composition de résine époxyde selon la revendication 12, **caractérisée en ce qu'**il s'agit d'une composant bicomposante constituée par
(i) un composant résine contenant au moins une résine époxyde et
(ii) un composant durcisseur contenant le durcisseur.

14. Composition durcie obtenue par le durcissement d'une composition de résine époxyde selon l'une quelconque des revendications 12 ou 13.

15. Article comprenant une composition durcie selon la revendication 14.
